# EUROPEAN PATENT SPECIFICATION

(11) **EP 1 622 532 B1**
(45) Date of publication and mention of the grant of the patent: **13.11.2013**
(21) Application number: 04759331.4
(22) Date of filing: 07.04.2004
(51) Int. Cl.: A61F 9/007, A61B 18/24

(54) **DEVICE FOR DACRYOCYSTORHINOSTOMY**
VORRICHTUNG FÜR DACRYOCYSTORHINOSTOMIE
DISPOSITIF POUR DACRYOCYSTORHINOSTOMIE

(30) Priority: 10.04.2003 US 411592
(43) Date of publication of application: 08.02.2006
(73) Proprietor: Biolitec Pharma Marketing Ltd., F.T. Labuan (MY)
(72) Inventor: TAMPLENIZZA, Paolo, I-20123 Milano (IT); CALDERA, Tiziano, Great Wilbraham Cambridgeshire CB1 5TA (GB); FONTANELLA, Walter, I-26013 Cremona (IT)
(74) Representative: Herrmann, Franz
(86) International application number: PCT/US2004/010973
(87) International publication number: WO 2004/091431

(56) References cited:
- WO-A1-02/36029
- WO-A1-98/23237
- DE-A1- 19 542 955
- DE-A1- 19 744 856
- DE-A1- 19 823 755
- US-A- 5 954 744
- US-A- 5 954 744
- US-A1- 2002 040 716
- US-B1- 6 251 103

## Description

### Background of the Invention

### 1. Field of the invention

The present invention relates to Dacryocystorhinostomy (DCR) treatments, particularly to introducers for DCR.

### 2. Information Disclosure Statement

DE 197 44 856 A1 discloses a device for microsurgery, that can be used for treating lachrymal ducts. The known device comprises a hand piece which is connected to the proximal end of a probe cannula. A drilling tool is axially moveable in the probe cannula. During insertion of the probe cannula, the distal end of the probe cannula remains open.

DE 195 42 955 A1 relates to an endoscope for treating lachrymal ducts. The endoscope comprises a cannula, in which an optical fiber can be inserted. The cross sections of the optical fiber and of the laser radiation fiber are smaller than the cross section of the inner channel of the cannula.

US 6 251 103 B1 refers to a transocular or periocular laser delivery system for treating eye diseases. The embodiments disclosed in US 6 251 103 B1 are not sealed to prevent fluids or other materials from getting inside the hollow outer tube.

Epiphora is defined as an overflow of tears, and occurs due to a disruption in the balance between tear production and drainage. Drainage is accomplished by a membranous channel known as the lachrymal drainage system. Reasons for improper tear drainage include poor lachrymal pump function due to a displaced punctum, eyelid laxity, weak orbicularis, or cranial nerve VII palsy, and anatomical obstruction of the drainage channel known as nasolachrymal drainage obstruction (NLDO). NLDO is relatively common, especially among the elderly, and although it rarely causes severe problems, constantly watering eyes due to improper drainage can be irritating and embarrassing and can cause impaired vision, excoriation of the skin and infections in the tear ducts.

The traditional method of treating NLDO involves external surgical removal of the stenosis. This is a lengthy procedure with considerable side effects such as postoperative pain. Recently, new systems have been developed to allow visualization of the nasolachrymal duct and allow treatments of stenosis by the application of small devices through the working channels of the system itself.

Instruments, such as lasers, which are a particularly interesting and important element in modem otorhinolaringoiatry, ophthalmology and general medical therapy, provide energy as a driving force and thus require accessories to allow the laser energy to be guided and delivered within the anatomical structure of the ear nose and throat area.

Treatment of NLDO involves surgical intervention known as Dacryocystorhinostomy (DCR), which basically consists of creating a new channel between the nasolachrymal duct and nasal cavity to facilitate tear drainage. Several established methods exist to perform DCR. Generally, this type of surgical intervention is classified as either external DCR or endoscopic DCR. External DCR entails making an incision on the side of the nose and removing a sufficient amount of bone to incorporate the lachrymal sac into the nose so that tears drain into the nasal cavity. (see U.S. Patent No. 5,345,948) External DCR requires skin incision and thus necessitates a longer recovery time than endoscopic DCR, and has a debatable success rate. Endoscopic DCR, which uses a laser to cut a passage from the lachrymal sac to the nasal cavity, is considered to be a first line treatment, in that it has a shorter recovery time, leaves no external scars and allows the use of local anesthetics.

Endoscopic DCR consists of a combination of both Endocanalicular and endoscopic nasal approaches, and thus generally requires the use of a number of instruments. It is either performed conventionally, such as in microdrilling, or is laser assisted.

U.S. Patent No. 5,345,948 provides a method of performing Translachrymal Laser Dacryocystorhinostomy involving insertion of a video endoscope and a bone cutting laser connected to a fiber optic bundle into the lachrymal sac. Illumination is provided by the fiber optic bundle and the laser is properly positioned with help from the video endoscope. The laser then is activated to create a tear-draining fistula. This method requires that two separate tools be inserted into the lachrymal sac at two separate points, namely the two puncta at the two canaliculi, which makes this method needlessly complex and thus prone to complication. A tube or stent may be placed in the fistula to keep it open after the procedure, although this would require a third direct insertion.

There are numerous devices and methods for facilitating the introduction of a stent to maintain an unobstructed drainage passage from the puncta and canaliculi to the nasal cavity. Such stents are generally used after DCR as a temporary device to prevent the newly created opening from occluding. They generally involve the introduction of a flexible tube, often by feeding one or more relatively rigid probes connected to the tube through the lachrymal system and drawing them through the nostrils. All such methods require tools for introduction of the stent that are separate from those tools used to introduce visualization or surgical instruments, and impose further trauma to the lachrymal tissue because instruments must be reinserted to position the stent.

U.S. Patent No. 5,062,831 describes a catheter for use in surgical correction of NLDO. This catheter, which is used to maintain the opening created between the lachrymal sac and the nasal cavity, has enlarged ends to avoid its slipping upward into the lachrymal sac or downward into the nose. This invention is limited for use with previously inserted slapstick tubing and requires an incision into both the lachrymal sac and nasal cavity to place the catheter.

U.S. Patent No. 2002/0107579 A1 describes a nasolachrymal duct tube used for introducing a stent into the nasolachrymal passage. Generally, a silicone tube is inserted through the superior and inferior canaliculi with a set of probes, and the tube is tied off in the nasal passage to form a stent that keeps the nasolachrymal passage open to facilitate drainage. In this invention, the probe or probes are made of a hollow tube that contains an opening at one end for connection of the flexible stent. Another opening is formed a specific distance from the stent opening to allow for an optical fiber or other illumination device to be inserted into the probe. This invention is limited to use with stent introduction, would generally be used after a DCR procedure, and does nothing to reduce the number of insertions and the related trauma to nasolachrymal tissue as a result of a DCR and insertion treatment. Furthermore, the illumination provided is limited to enhancing direct visualization through the nasal cavity, and would thus not provide interior visualization and would not be useful during a DCR procedure.

U.S. Patent No. 2002/0151960 describes a monocanalicular stent for maintaining a newly constructed drainage channel. The stent consists of a preferably silicone tube connected to a plug located at the tube's proximal end that is shaped to fit the punctum, and a stylet inserted into the tube to direct and advance the tube to the newly constructed drainage channel. The stylet is removed after the tubing and plug are in position. Like the above patent, this device must still be newly inserted into the nasolachrymal system after the DCR treatment, and thus inflicts further trauma to the tissue.

There remains a need for a device and method for laser assisted DCR that is minimally invasive and requires minimal direct insertions, while preserving the ability to visualize the treatment area, create a fistula and insert an intubation tube or stent. The present invention addresses these needs.

### Objectives and Brief Summary of the Invention

It is an object of the present invention to provide a device for a minimally traumatic treatment of nasolachrymal drainage obstruction.

It is another object of the present invention to provide a device for laser assisted dacryocystorhinostomy (DCR) that is minimally traumatic and requires only one traumatic insertion of instruments.

It is a further object of the present invention to provide an introducer for laser assisted DCR that eliminates the need for repeated traumatic insertions of instruments and provides for access of an intubation tube or stent.

It is yet another object of the invention to provide a DCR Introducer for Laser treatments, that can be applied to any existing clinical environment without the need to modify additional instruments or setups, as well as to provide a fully functional environment itself which can be extended by state of the art ear nose and throat elements by simple compatibility means.

Briefly stated, the present invention provides an introducer and a system for treatment of nasolachrymal drainage obstruction (NLDO). The introducer comprises a semi-rigid hollow outer tube and an atraumatic inner mandril. The inner mandril has a rounded distal end to aid insertion and reduce trauma during insertion. The introducer is inserted into the lachrymal sac section. The atraumatic inner mandril is removed and one or more optical fibers or fiber bundles are inserted for illumination to determine proper position and for ablating a drainage channel. The fiber or bundle is then removed and a DCR intubation set can then be introduced to maintain the drainage channel. One advantage of the present invention is that all aspects of the procedure can be performed through the introducer, thus only requiring a single insertion point and reducing trauma to the nasolachrymal duct, and reducing the complexity of the treatment and the risk of complication or infection. Another advantage of the present invention is that it can be used with many of pre-existing intubation tubes used for current DCR procedures, thus the present device can easily and cost effectively be introduced as a first line procedure.

The above, and other objects, features and advantages of the present invention will become apparent from the following description read in conjunction with the accompanying drawings.

### Brief Description of Figures

Fig. 1- An illustration of the nasolachrymal tear drainage system.
Fig. 2 - A cross sectional view of a preferred embodiment of the DCR Introducer.
Fig. 3 - An illustration of the introducer as used in DCR and stent insertion.

### Detailed Description of Preferred Embodiments

One aspect of the present invention is an atraumatic introducer for DacryoCystoRhinostomy (DCR) laser treatment that provides simultaneous access to the lachrymal sac for illumination means and ablation means and for many standard intubation sets. Other aspects of the present invention include a DCR system including the introducer, a radiation source and optical fiber or fiber bundle, and an intubation set.

A general problem in laser DCR procedures is the difficulty encountered in accessing the newly created opening between the lachrymal sac and the medium meatus in order to insert the intubation set or stent that serves to preserve the opening after treatment. The medium meatus is under the medium turbinate, which is one of three principal bones that protrude into the nasal cavity. The present invention solves this problem by allowing numerous operations, namely the laser DCR treatment and the intubation set insertion, as well as any other needed operations such as visualization, to be conducted without causing excessive trauma to the patient.

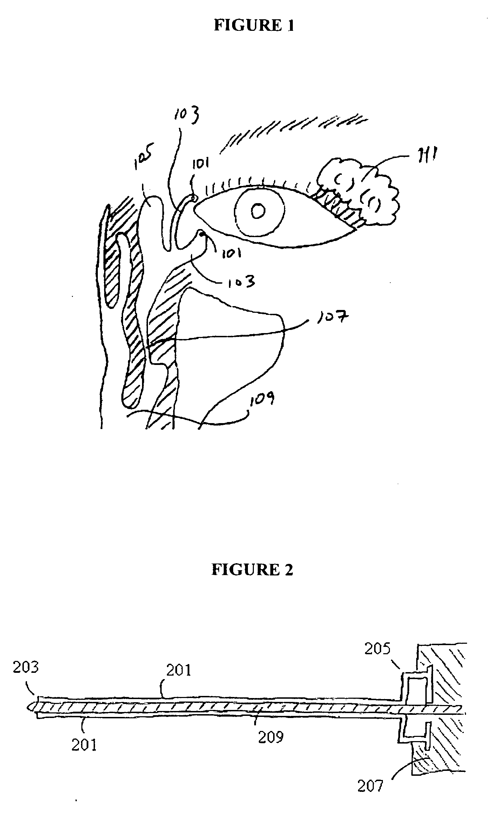

The following terms used in this disclosure are defined below. **Figure 1** illustrates various parts of the nasolachrymal tear drainage system that are referred to below. "Puncta" (101) are small openings located at the internal corner of the eyelids that function as a drain for tears. There is an upper and a lower punctum located on the respective eyelids. The "canaliculi" (103) serve to connect the puncta to the lachrymal sac and allow tears to drain from the eye to the lachrymal sac. The "lachrymal sac" **(105)** is a large area that collects tears. Its upper end is rounded and its lower end extends into the nasolachrymal duct. The "nasolachrymal duct" **(107)** is an osteo-membranous canal, which extends from the lower part of the lachrymal sac to the nasal cavity **(109).** Tears are produced in the lachrymal gland (**111**), drain into the lachrymal sac via the puncta and canaliculi, continue on through the lachrymal duct and ultimately drain into the nose.

"Proximal end" refers to the end of the introducer to which a handpiece is attached and through which various instruments are inserted. "Distal end" refers to that end of the introducer that is inserted into the punctum in typical DCR applications and which is advanced to the nasolachrymal duct. These references also apply to all components and instruments that are advanced through the tube of the introducer.

Further it is a matter of significant importance that an enlargement of the treatment area is compatible with existing intubation sets in order to keep the costs reasonable.

There are numerous advantages inherent in the new DCR Introducer for Laser treatment. First, the small size of the DCR Introducer (preferably less than 1 mm in diameter) allows for atraumatic introduction of an optical fiber or fiber bundle within the nasolachrymal duct to reach the blockage. Another advantage is that the DCR treatment can be performed under safer conditions by the use of laser energy. To make the opening without destroying surrounding tissue, the laser source safely delivers the laser energy within the nasolachrymal duct following determination of a clinical protocol for power density to the level indicated. Furthermore, the introducer itself enhances the safety of the laser treatment by guiding the optical fiber, which can be very fine (for example 220 microns), to the desired location in the lachrymal sac without the possibility of puncturing other tissue or following the wrong path. It also serves to protect the lachrymal system in the event of accidental irradiation while the fiber is advanced toward the treatment area and prevent fiber fractures.

A third major advantage is that an intubation set for maintaining the opening created by the laser can now be inserted into the opening by the user through the DCR Introducer while avoiding successive trauma to the nasolachrymal duct and the risk of difficulties in finding the new created pathway. Many intubation sets are known and used, and can be newly inserted into the punctum through the outer tube after the optical fiber is removed. Insertion of a stent through the punctum without the present invention requires that the user insert the stent and search for the newly created way, which can cause additional pain and trauma to the patient. The present invention avoids this additional trauma because the introducer tube has already been inserted. Thus the stent and other instruments may be introduced without additional trauma. With the present invention, the patient need only experience one traumatic insertion and removal although numerous instruments including the stent are introduced.

Therefore it is the aim of the present invention to provide a small DCR Introducer that fulfills all requirements setup by clinical use and that can be operated with simple means. Another subject of the present invention is to utilize the safety advantages inherent in delivering energy to the treatment zone with laser fibers while providing access (or multiple access during the same treatment) for positioning a nasolachrymal duct intubation set. This solves all of the mentioned problems, as the introducer can be compatible with existing intubation sets.

The present invention discloses an introducer consisting of a hollow tube of sufficiently small diameter to be inserted through the canaliculi into the lachrymal sac without incision and of sufficient length to reach the lachrymal duct through the lachrymal sac. In a preferred embodiment, the introducer tube is made from a rigid or semi-rigid material. The introducer also contains an inner mandril that is located within the tube during insertion of the introducer into the lachrymal sac. Both the hollow tube and the inner mandril are preferably constructed from an autoclavable metallic material. The inner mandril also contains a round shaped distal end which aids insertion of the introducer by reducing trauma during insertion. In addition to aiding insertion, the inner mandril serves to prevent fluids or other materials from getting inside of the introducer tube and potentially causing an obstruction or other problems during insertion of subsequent instruments. In a preferred embodiment, the inner mandril is constructed from the same material as the introducer tube. The introducer is preferably connected to a handpiece for ease of use.

After the introducer is in place within the lachrymal sac or in the nasolachrymal duct, numerous instruments may be inserted into the tube without additional trauma to the tissue. An optical fiber or optical fiber bundle may be inserted for delivery of ablative laser radiation. To ensure that the distal end of the introducer is in proper position, an endoscope may be inserted prior to insertion of the laser delivery fiber(s) to visualize the area, by means of transillumination. Alternatively, means to visualize the area and fiber(s) for ablation may be incorporated into a single bundle to further simplify the process.

Other advantages to utilizing lasers in the present invention include shorter treatment times and more simplified treatments due to lasers' dual ability to illuminate and cut tissue. Furthermore, at some wavelengths, lasers can ablate/cut and cauterize simultaneously, such as at 980 nm. The importance of the ability to simultaneously ablate and cauterize tissue is that this ability results in a better view of the treatment area because bleeding is minimized. Thus, for such wavelengths, illumination and/or visualization means can be quickly and efficiently utilized to guide the procedure.

The introducer of the present invention is not limited to use with laser assisted occlusion reduction methods. Mechanical methods such as microdrilling may also be used, as well as electrosurgical instruments. The use of the present invention with such methods retains the advantage of reducing trauma to lachrymal system tissue.

After the drainage opening is created, an intubation tube or stent may be inserted into the opening to prevent closure. The intubation tube can be inserted through the introducer to avoid further traumatic reinsertion of instruments, thus again simplifying the procedure and reducing trauma. The outside tube of the introducer is of sufficient diameter to accommodate many devices and methods for intubation.

A preferred method of using the present invention is performed as follows. The DCR introducer is inserted through the lower or upper punctum and the lower or upper canaliculus to reach the lachrymal sac section or duct. The atraumatic inner part of the introducer is then removed and an optical fiber inserted through the introducer itself so that the distal end of the fiber is advanced beyond the distal end of the introducer tube. A laser aiming beam can then be activated to check proper introducer position.

When the optical fiber is properly positioned, laser energy is applied to achieve an opening between the nasolachrymal duct and medium meatus, which allows the introducer to be moved into a position visible through the nasal cavity. The laser fiber is then removed, the introducer is advanced through the opening, and the DCR Intubation set is inserted through the introducer and placed within the lachrymal ways.

**Figure 2** illustrates a preferred embodiment of the DCR introducer. Outer tube **201,** which is of a sufficiently small diameter to be inserted into the nasolachrymal duct through the punctum without an incision, has an opening at distal end **203** and a larger opening **205** at the proximal end of tube **201** to ease insertion of various instruments. Handpiece **207** is also located at the proximal end of tube **201.** Mandril **209** is inserted into tube **201** prior to insertion of the handpiece into the nasolachrymal duct.

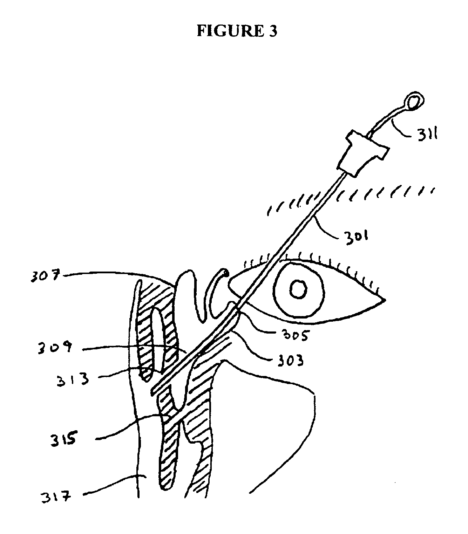

**Figure 3** shows the basic elements of the DCR procedure and the application of the introducer as the basis of this invention. Introducer **301** is inserted into lower canaliculus **303** via lower punctum **305,** and is further advanced through lachrymal sac **307** into nasolachrymal duct **309.** Atraumatic inner mandril **311** is removed after the distal end of introducer **301** is in position. An optical fiber connected to a suitable laser source is then inserted into introducer until the distal end of the fiber extends from the distal end of introducer **301.** The radiation is activated until opening **313** has been formed through lachrymal bone **315** and now connects nasolachrymal duct **309** and nasal cavity **317.**

The present invention is further illustrated by the following examples, but is not limited thereby.

### Example 1:

A handpiece utilizing the present invention features an introducer that consists of a metal tube with an outside diameter of 0.9 mm and an inside diameter of at least 0.7 mm. A solid cylindrical mandril is located inside the tube and has a diameter of less than 0.7 mm. The mandril has a pull ring at the proximal end to facilitate removal after the introducer is inserted.

A 980 nm diode laser is coupled to an optical fiber with a preferred diameter of 200 microns. Alternatively, an 810 nm diode laser is coupled to the fiber or both an 810 nm and 980 nm laser can be simultaneously coupled to the fiber. After insertion of the introducer, the mandril is removed and the 200 micron fiber is inserted into the tube. The diode laser is then activated to ablate tissue and create a drainage opening.

After the drainage opening is completed, the introducer is advanced through the opening and the fiber is atraumatically withdrawn. After the fiber is withdrawn, using the introducer like a mark, an endoscopic intranasal enlargement of the newly formed opening end can be performed, if required. An intubations set is then inserted through the introducer, and the introducer tube is then withdrawn. The other tip of the stent is then introduced in the upper punctum in order to reach the nose through the upper canaliculum and the newly formed nasolachrymal duct, in order to form the loop. The two tips of the intubations set are, as normal, anchored in the nose with a knot. The set is left in place for a period of time, for example up to 5-6 months, to prevent the rerouted canal from closing up.

Having described preferred embodiments of the invention with reference to the accompanying drawings, it is to be understood that the invention is not limited to the precise embodiments, and that various changes and modifications may be effected therein by those skilled in the art without departing from the scope of the invention as defined in the appended claims.

## Claims

1. A device for minimally traumatic treatment of obstructions in the lachrymal drainage system comprising:
a hollow outer tube (201) capable of being inserted into said lachrymal apparatus without the need for an incision; and
an atraumatic inner rod (209, 311) inserted into said outer tube (201), wherein said inner rod (209, 311) is removable after insertion of said outer tube (201) into said lachrymal drainage system, and wherein means for treating said obstruction are insertable without causing further trauma to said lachrymal drainage system
**characterized in that**
diameters of the inner rod (209, 311) and of the hollow outer tube (201) are dimensioned such that the inner rod (209, 311) is configured to seal the hollow outer tube (201) upon insertion, and serves to prevent fluids and other materials from getting inside the hollow outer tube (201).

2. The device according to claim 1,
wherein said outer tube (201) is rigid or semi-rigid.

3. The device according to claim 1,
wherein said outer tube (201) and said inner rod (209, 311) are made from an autoclavable metallic material.

4. The device according to claim 1,
wherein a distal end of said inner rod (209, 311) has a rounded shape to reduce trauma during insertion of the device.

5. The device according to claim 1,
wherein said means for treating said obstruction is selected from the group consisting of a radiation treatment device, a mechanical treatment device and an electro-surgical treatment device.

6. The device according to claim 1,
wherein said outer tube (201) has a maximum outer diameter of 1 mm.

7. A system for minimally traumatic treatment of obstructions in the lachrymal drainage system comprising:
the device of claim 1 comprising a hollow outer tube (201) and an inner rod (209, 311);
a radiation source; and
delivery means connected to said source to deliver radiation from said source to a location in the lachrymal drainage system sufficient to create a drainage channel, wherein said delivery means is atraumatically insertable into said outer tube (201) after insertion of said device.

8. The system according to claim 7,
wherein said radiation source is a laser.

9. The system according to claim 7,
wherein said radiation source emits radiation at a wavelength selected from the group consisting of 810 nm and 980 nm.

10. The system according to claim 7,
wherein said delivery means is selected from the group consisting of an optical fiber and an optical fiber bundle.

11. The system according to claim 7,
further comprising a flexible stent that is insertable into said hollow tube after withdrawal of said delivery means to maintain said drainage channel.

12. The system according to claim 8 arranged to be operated for a minimally traumatic treatment of obstructions in the lachrymal drainage system, comprising the steps of:
a. inserting an atraumatic inner rod (209, 311) into a hollow outer tube (201);
b. inserting said outer tube (201) into a lachrymal duct, wherein a distal end of said outer tube (201) is in proximity to a predetermined location in said lachrymal duct;
c. removing said inner rod (209, 311);
d. the massive inner rod (209, 311) preventing fluids and other materials from getting inside the hollow outer tube (201), wherein diameters of the massive inner rod (209, 311) and of the hollow outer tube (201) are dimensioned such that the inner rod (209, 311) is configured to seal the hollow outer tube (201) upon insertion;
e. inserting ablative means to emit ablative laser radiation into said outer tube (201) until a distal end of said ablative means extends from said distal end of said outer tube (201); and
f. activating said ablative means to emit ablative laser radiation to create an opening between said lachrymal duct and a nasal cavity.

13. The system according to claim 12,
arranged for a treatment comprising the additional steps of:
g. advancing said distal end of said outer tube (201) through said opening until said distal end of said outer tube (201) is visible through said nasal cavity;
h. inserting an intubation tube through said outer tube (201) so that said intubation tube is located in said opening; and
i. removing said outer tube (201) so that said intubation tube remains to support said opening.

14. The system according to claim 12,
wherein said step of inserting said outer tube (201) into said lachrymal duct is accomplished by inserting said tube (201) into a lower punctum.

15. The system according to claim 12,
wherein said means to emit ablative laser radiation is at least one optical fiber connected to at least one laser radiation source.

## Patentansprüche

1. Vorrichtung für die minimal-traumatische Behandlung von Beschränkungen des lachrymalen Drainagesystems mit:
einer hohlen äußeren Röhre (201), die dazu in der Lage ist, ohne Einschnitt in die lachrymale Vorrichtung eingebracht zu werden; und
einem atraumatischen inneren Stab (209, 311), der in die äußere Röhre (201) eingebracht ist, wobei der innere Stab (209, 311) nach dem Einführen der äußeren Röhre (201) in das lachrymale Drainagesystem entfernbar ist und wobei Mittel zur Behandlung der Beschränkung einführbar sind, ohne ein weiteres Trauma im lachrymalen Drainagesystem hervorzurufen,
**dadurch gekennzeichnet, dass**
Abmessungen des inneren Stabs (209, 311) und des hohlen äußeren Rohres (201) derart bemessen sind, dass der innere Stab (209, 311) dazu eingerichtet ist, das hohle äußere Rohr (201) beim Einführen abzudichten, und dazu dient, Fluide und andere Materialien davon abzuhalten, ins Innere des hohlen äußeren Rohres (201) zu gelangen.

2. Vorrichtung nach Anspruch 1,
bei der das äußere Rohr (201) starr oder halbstarr ist.

3. Vorrichtung nach Anspruch 1,
bei der das äußere Rohr (201) und der innere Stab (209, 311) aus einem dampfsterilisierbaren metallischen Material hergestellt sind.

4. Vorrichtung nach Anspruch 1,
bei der ein distales Ende des inneren Stabs (209, 311) eine abgerundete Form aufweist, um das Trauma beim Einführen des Geräts zu verringern.

5. Vorrichtung nach Anspruch 1,
bei der das Mittel zur Behandlung der Beschränkung aus der Gruppe ausgewählt ist, die aus einem Strahlungsbehandlungsgerät, einem mechanischen Behandlungsgerät und einem elektrochirurgischen Behandlungsgerät besteht.

6. Vorrichtung nach Anspruch 1,
bei der das äußere Rohr (201) einen maximalen äußeren Durchmesser von 1 mm aufweist.

7. System zur miminal-traumatischen Behandlung von Beschränkungen im lachrymalen Drainagesystem mit:
der Vorrichtung nach Anspruch 1 mit einem hohlen äußeren Rohr (201) und einem inneren Stab (209, 311);
einer Strahlungsquelle; und
mit der Quelle verbundene Zuführmittel, um zum Erzeugen eines Drainagekanals ausreichende Strahlung von der Quelle zu einem Ort in dem lachrymalen Drainagesystem zu führen, wobei die Zuführmittel nach dem Einbringen der Vorrichtung atraumatisch in die äußere Röhre (201) einbringbar sind.

8. System nach Anspruch 7,
bei dem die Strahlungsquelle ein Laser ist.

9. System nach Anspruch 7,
bei dem die Strahlungsquelle Strahlung bei einer Wellenlänge emittiert, die aus der Gruppe bestehend aus 810 nm und 980 nm ausgewählt ist.

10. System nach Anspruch 7,
bei dem die Zuführmittel von der Gruppe ausgewählt sind, die aus einer optischen Faser und einem optischen Faserbündel besteht.

11. System nach Anspruch 7,
das ferner einen flexiblen Stent aufweist, der nach einem Zurückziehen der Zuführmittel in das hohle Rohr einführbar ist, um den Drainagekanal zu erhalten.

12. System nach Anspruch 8, das für den Betrieb im Zusammenhang mit einer minimal-traumatischen Behandlung von Beschränkungen im lachrymalen Drainagesystem eingerichtet ist, das die Schritte umfasst:
a. Einbringen eines atraumatischen inneren Stabs (209, 311) in ein hohles äußeres Rohr (201);
b. Einbringen des äußeren Rohrs (201) in einen lachrymalen Kanal, wobei ein distales Ende des äußeren Rohrs (201) sich in der Nähe eines vorbestimmten Ortes in dem lachrymalen Kanal befindet;
c. Entfernen des inneren Stabs (209, 311);
d. durch den massiven inneren Stab (209, 311) werden Fluide und anderes Material davon abgehalten, in das hohle äußere Rohr (201) zu gelangen, wobei die Durchmesser des massiven inneren Stabs (209, 311) und des hohlen äußeren Rohrs (201) derart bemessen sind, dass der innere Stab (209, 311) dazu eingerichtet ist, das hohle äußere Rohr (201) beim Einbringen abzudichten;
e. Einbringen von ablativen Mitteln für die Emission von ablativer Laserstrahlung in das äußere Rohr (201) bis sich ein distales Ende der ablativen Mittel vom distalen Ende des äußeren Rohrs (201) erstreckt; und
f. Aktivieren der ablativen Mittel, um ablative Laserstrahlung zu emittieren, um eine Öffnung zwischen dem lachrymalen Kanal und dem Nasenhohlraum zu schaffen.

13. System gemäß Anspruch 12,
das für eine Behandlung mit den zusätzlichen Schritten eingerichtet ist:
g. Vorschieben des distalen Endes des äußeren Rohres (201) durch die Öffnung bis das distale Ende durch den Nasenhohlraum sichtbar ist;
h. Einführen eines Intubationsrohres durch das äußere Rohr (201), so dass sich das Intubationsrohr in der Öffnung befindet; und
i. Entfernen des äußeren Rohrs (201), so dass das Intubationsrohr zum Stützen der Öffnung verbleibt.

14. System nach Anspruch 12,
bei dem der Schritt des Einbringens des äußeren Rohrs (201) in den lachrymalen Kanal durch Einführen des Rohrs (201) in ein unteres Tränenpünktchen ausgeführt wird.

15. System nach Anspruch 12,
bei dem die Mittel zum Emittieren von ablativer Laserstrahlung wenigstens eine optische Faser ist, die mit wenigstens einer Laserstrahlungsquelle verbunden ist.

## Revendications

1. Dispositif pour le traitement minimalement traumatique des obstructions dans le système d'écoulement lacrymal comprenant:
un tube externe creux (201) pouvant être inséré dans ledit appareil lacrymal sans avoir besoin d'incision; et
une tige interne atraumatique (209, 311) insérée dans ledit tube externe (201), dans lequel ladite tige interne (209, 311) peut être retirée après l'insertion dudit tube externe (201) dans ledit système d'écoulement lacrymal, et dans lequel des moyens pour traiter ladite obstruction peuvent être insérés sans provoquer de traumatisme supplémentaire audit système d'écoulement lacrymal,
**caractérisé en ce que**:
des diamètres de la tige interne (209, 311) et du tube externe creux (201) sont dimensionnés de sorte que la tige interne (209, 311) est configurée pour sceller le tube externe creux (201) suite à l'insertion, et sert à empêcher l'entrée des fluides ou d'autres matières à l'intérieur du tube externe creux (201).

2. Dispositif selon la revendication 1,
dans lequel ledit tube externe (201) est rigide ou semi-rigide.

3. Dispositif selon la revendication 1,
dans lequel ledit tube externe (201) et ladite tige interne (209, 311) sont réalisés à partir d'un matériau métallique pouvant passer à l'autoclave.

4. Dispositif selon la revendication 1,
dans lequel une extrémité distale de ladite tige interne (209, 311) a une forme arrondie pour réduire le traumatisme pendant l'insertion du dispositif.

5. Dispositif selon la revendication 1,
dans lequel lesdits moyens pour traiter ladite obstruction sont choisis dans le groupe comprenant un dispositif de traitement par rayonnement, un dispositif de traitement mécanique et un dispositif de traitement électrochirurgical.

6. Dispositif selon la revendication 1,
dans lequel ledit tube externe (201) a un diamètre externe minimum de 1 mm.

7. Système pour le traitement minimalement traumatique des obstructions dans le système d'écoulement lacrymal, comprenant:
le dispositif selon la revendication 1, comprenant un tube externe creux (201) et une tige interne (209, 311);
une source de rayonnement; et
des moyens de distribution raccordés à ladite source pour distribuer le rayonnement de ladite source à un emplacement dans le système d'écoulement lacrymal suffisant pour créer un canal d'écoulement, dans lequel lesdits moyens de distribution sont insérés de manière atraumatique dans ledit tube externe (201) après l'insertion dudit dispositif.

8. Système selon la revendication 7,
dans lequel ladite source de rayonnement est un laser.

9. Système selon la revendication 7,
dans lequel ladite source de rayonnement émet le rayonnement à une longueur d'onde choisie dans le groupe comprenant 810 nm et 980 nm.

10. Système selon la revendication 7,
dans lequel lesdits moyens de distribution sont choisis dans le groupe comprenant une fibre optique et un faisceau de fibres optiques.

11. Système selon la revendication 7,
comprenant en outre un stent flexible qui peut être inséré dans ledit tube creux après le retrait desdits moyens de distribution pour maintenir ledit canal d'écoulement.

12. Système selon la revendication 8, agencé pour être actionné pour un traitement minimalement traumatique des obstructions dans un système d'écoulement lacrymal, comprenant les étapes consistant à:
a. insérer une tige interne (209, 311) atraumatique dans un tube externe creux (201);
b. insérer ledit tube externe (201) dans un conduit lacrymal, dans lequel une extrémité distale dudit tube externe (201) est à proximité d'un emplacement prédéterminé dans ledit conduit lacrymal;
c. retirer ladite tige interne (209, 311);
d. la tige interne (209, 311) massive empêchant des fluides et d'autres matières d'entrer à l'intérieur du tube externe creux (201), dans lequel des diamètres de la tige interne (209, 311) massive et du tube externe creux (201) sont dimensionnés de sorte que la tige interne (209, 311) est configurée pour sceller le tube externe creux (201) suite à l'insertion;
e. insérer des moyens d'ablation pour émettre un rayonnement laser d'ablation dans ledit tube externe (201) jusqu'à ce qu'une extrémité distale desdits moyens d'ablation s'étende à partir de ladite extrémité distale dudit tube externe (201); et
f. activer lesdits moyens d'ablation pour émettre le rayonnement laser d'ablation afin de créer une ouverture entre ledit conduit lacrymal et une cavité nasale.

13. Système selon la revendication 12,
agencé pour un traitement comprenant les étapes supplémentaires consistant à:
g. faire avancer ladite extrémité distale dudit tube externe (201) par ladite ouverture jusqu'à ce que ladite extrémité distale dudit tube externe (201) soit visible à travers ladite cavité nasale;
h. insérer un tube d'intubation à travers ledit tube externe (201) de sorte que ledit tube d'intubation soit positionné dans ladite ouverture; et
i. déplacer ledit tube externe (201) de sorte que ledit tube d'intubation demeure pour supporter ladite ouverture.

14. Système selon la revendication 12,
dans lequel ladite étape consistant à insérer ledit tube externe (201) dans ledit conduit lacrymal est réalisée en insérant ledit tube (201) dans un point lacrymal inférieur.

15. Système selon la revendication 12,
dans lequel lesdits moyens pour émettre le rayonnement laser d'ablation sont au moins une fibre optique raccordée à au moins une source de rayonnement laser.
